# EUROPEAN PATENT APPLICATION

(11) **EP 1 650 566 A1**
(43) Date of publication of application: **26.04.2006**
(21) Application number: 05022627.3
(22) Date of filing: 18.10.2005
(51) Int. Cl.: G01N 33/58, G01N 33/533

(54) **Ligand-receptor tracking assays**

(30) Priority: 25.10.2004 EP 04105285
(71) Applicant: F.HOFFMANN-LA ROCHE AG, 4070 Basel (CH)
(72) Inventor: Apfel, Christian, 79618 Rheinfelden (DE); Enderle, Thilo, 79618 Rheinfelden (DE); Jensen Zoffmann, Sannah, 4058 Basel (CH); Moser, Mireille, 22149 Hamburg (DE)

(57) **Abstract**

The present invention provides an image-based method of screening for compounds based on the measurement of internalization of a fluorescently labeled ligand. It further provides a multiplex method for high content screening of compounds wherein at least one cellular parameter is measured.

## Description

High content screening has recently emerged as a new approach compatible with high throughput screening in drug development. High content screening is a technology platform designed to define the temporal and spatial activities of genes, proteins, and other cellular constituents in living cells.

One central component in a reader for high content screening is a microscope which is adapted to image acquirement of samples in microtiter plates. The reader is equipped with software that allows automated image acquirement and analysis. Analysis can be based on luminescence and/or bright field images.

One type of activity which is of interest for drug screening is agonist-induced internalization of ligand-receptor complexes. Agonist-induced internalization and recycling has been shown for the PTH (parathyroid hormone)-receptor by imaging of PTH receptor fused to Green Fluorescent Protein (GFP) (Conway et al., 2001, J. Cell. Physiol. 189, p. 341-355). Schlag et al. described internalization of 5HT2c-GFP, whereby internalization was detected in one cell line using an ArrayScan reader. Ca²⁺ flux was determined separately by a different assay, FLIPR (Schlag et al., 2004, J. Pharmacol. Exp. Ther. 310, 865-870). Gao et al., described a manual quantification of internalization of a GFP tagged MC4 receptor which is not suitable for large scale screening purposes (Gao et al., 2003, J. Pharmacol. Exp. Ther. 307, 870-877). Giuliano et al. disclosed multiparameter high content screening within the same population of single cells. This reference does not disclose ligand tracking or multiplexing with more than one type of cells (Giuliano et al., 2003, Assay Drug Dev. Technol. 1(4), 565-577).

The present invention provides a multiplex method for high content screening of compounds. Multiplexing describes a method where more than one independent parameter is acquired as the readout. With High Content Screening, the image based analysis directly assigns a certain measured optical signal to the individual identified cell. This allows multiplexing both for multiple events within the individual cells, and for multiple events in multiple cells that express different receptors. In one embodiment, it allows multiplexing for multiple events in a heterogeneous mix of cells, where subpopulations may respond differently and can be distinguished by optical means. In another embodiment, it allows multiplexing of multiple events in subpopulations of cells which may respond differently, whereby the subpopulations can be differentiated from each other.

The present method thus allows screening of compounds by measuring their effects on multiple cellular parameters in multiple cell populations.

Accordingly, in the multiplexing method of the present invention, at least one cellular parameter of at least one single cell or at least one subpopulation of cells or a heterogeneous population of cells which express at least one target receptor, are measured optically in the presence or absence of a compound. For this method, cell lines, stably or transiently transfected cells or primary cell cultures can be used which express one or more target receptors. Preferably, at least two cellular parameters are measured. More preferably, at least three cellular parameters are measured. In another preferred embodiment, at least four different parameters are measured.

The term "optically measured" and "measured" is understood to mean a quantitative determination of an optical signal.

It is preferable, for the method hereinbefore described, that said parameter or parameters are measured in at least two single cells which express different target receptors, or at least two different subpopulations of cells which express different target receptors. Said parameter or parameters can also be measured in at least three single cells which express different target receptors, or at least three different subpopulations of cells which express different target receptors.

Any one of the target receptors of the method described herein may be any type of receptor that is internalized following stimulation by an agonist. Preferably, said target receptor is a G-protein coupled receptor (GPCR). More preferably, said receptor is selected from the group consisting of Somatostatin receptor 5 (SSTR-5) (Seq ID No. 1), Growth hormone secretagogue receptor 1a (GHSR-1a) (Seq ID No. 2), Vasopressin-lb receptor (Seq ID No. 3), or Neurokinin 3 receptor (NK3R) (Seq ID No. 4). Cells used in the method disclosed herein can be either cells naturally expressing a target receptor, or cell which are transfected either stably or transiently with a target receptor.

In another preferred embodiment, a mixture of cells expressing either one of at least two target receptors is used. In a more preferred embodiment, subpopulations of cells are analyzed by the multiplex method. A subpopulation of cells can consist of one or more cells. In another preferred embodiment, subpopulations of cells may, e.g., be differentiated by their position in a single well or by expression of or staining with different dyes. In a further more preferred embodiment, transiently or most preferably stably transfected cells are used. A subpopulation of cells consists of a single cell or more than one cell.

The cellular parameters may be any cellular parameter that can be detected optically. As non-limiting examples, these parameters may be:

The position of subpopulations of cells, or of single cells, whereby the cells may be independently marked with fluorescent or non-fluorescent dyes, including transfection with fluorescent proteins (e.g. GFP or others known in the art); Ca2+ flux in cells; translocation of labeled proteins from one cellular compartment to another, wherein said label can be detected optically; degradation of proteins; detection of gene expression using reporter genes; detection of cell apoptosis; internalization of receptors or ligands or small molecular agonists which induce internalization into cells.

The present invention discloses a high content ligand-receptor tracking method optimized for drug screening on cells. The method is based on an image-based tracking of the local concentration and subcellular localization of a labeled ligand and/or receptor. The present invention makes it possible to correlate receptor-ligand tracking with other cellular parameters, which is achieved by multiplexing. Preferably, ligand-receptor internalization is being tracked by the present method.

Receptor desensitization is a mechanism that allows the cell to regulate the response to stimulation by an extracellular agonist. Desensitization is important for the regulation of the sensitivity of cells towards extracellular stimuli, since it allows a rapid attenuation of the induced receptor activity, preparing the cell for subsequent stimulation with similar or different agonists using same cellular signaling pathways. A second important function of desensitization is modulation of the sensitivity by consecutive exposures to an agonist. For GPCRs, the attenuation of the signal involves phosphorylation of the intracellular domains, followed by a physical removal of receptor proteins from the cell surface. This internalization of receptors takes place by formation of vesicles from the extracellular membrane, containing membrane embedded phosphorylated receptor and ligand located in the vesicular lumen. Subsequently, the receptor can be degraded in lysosomes or resensitized and recycled back to the cell surface.

Assays that screen for compounds affecting receptor internalization will be able to identify receptor ligands with a different pharmacological profile, which act as competitors in classical competition binding assays. In addition, compounds able to target other steps in the internalization mechanism can also be detected in ligand-receptor tracking assays.

Internalization of the ligand or receptor is tracked by quantitative imaging of internalization and accumulation of the signal provided by a label which can be measured optically in intracellular compartments, and which can be correlated to other cellular parameters which are measured in the same cells in parallel. This method is superior to ligand tracking methods in the prior art such as described by Nouel et al, 1997, Endocrinology 138, 296-306; or Rocheville et al., 2000, J. Biol. Chem. 275, 7862-7869. In particular, a signal sufficiently strong to do a multiplex drug screening including ligand-receptor tracking could only be obtained with image-based detection. Even though, in the case of a labeled agonist, the internalized agonist is eventually degraded or dissociated from the receptor, it will remain concentrated in intracellular compartments for a sufficient period of time to give a direct proportional measure for the amount of receptors present at the cell surface when internalization was induced.

Thus, in a preferred embodiment of the present invention, at least one of at least two cellular parameters measured is receptor-ligand internalization, wherein said ligand-receptor internalization is tracked by optical detection of a labeled ligand or labeled receptor.

In this embodiment of the present invention, an agonist for a target receptor is provided which induces receptor internalization. Receptor-ligand internalization can be detected either by tracking a labeled ligand (agonist), or tracking a labeled target receptor, wherein said label can be detected optically. The label may be attached to the ligand or target receptor either covalently or non-covalently, and optical detection may be direct or indirect. In a preferred embodiment, the label is a luminophore. It is also preferable that said labeled receptor is not a receptor to which a fluorescent protein, such as GFP is fused.

Labels may include luminophores, preferably fluorophores, which are covalently grafted onto the target receptor or ligand, of fluorescent proteins fused to the ligand, such as e.g. GFP. Alternatively, labels may also be antibodies which bind said target receptor without inducing activation. Said antibodies may then be tracked through a label that is attached to them. Such labels may be any label that can be detected optically, either directly or indirectly, in live or fixed cells. Adie et al. disclosed the tracking of internalized GPCRs to which a tag was fused which can be bound with a specific antibody, with said antibody to which a pH sensitive, optically detectable tag was bound (Adie et al., 2003, Assay Drug Dev. Technol. 1, 251-259). Other labels include the biotin-streptavidin system, or any other system in which a luminophore, or more preferably a fluorophore is bound to an agent which can bind in a non-covalent manner to the ligand or agonist or receptor or a tag fused or linked to them. Suitable detection systems also include Lanthanide Chelate detection systems. In a preferred embodiment of the present invention, a ligand is tracked which is labeled with a fluorophore. This fluorophore-labeled ligand is then incubated with cells expressing the target receptor in the presence or absence of said compounds. As described hereinbefore, said cells may be cell lines or primary cell cultures which express one or more target receptors. In one preferred embodiment, a mixture of cells expressing either one of at least two target receptors is used. In a more preferred embodiment, subpopulations of cells expressing at least one target receptor are analyzed by the multiplex method. In another preferred embodiment, subpopulations of said cells may, e.g., be differentiated by their position in a single well. In a most preferred embodiment, single cells are analyzed by the multiplex method of the present invention.

Receptor agonists can either be small molecules that were previously identified to act as an agonist for a given target receptor and to induce receptor internalization, or a natural ligand, preferably a peptide or a protein, or a fragment thereof which can act as an agonist and mediate receptor internalization. The term "agonist" and "ligand" are used interchangeably and both include natural ligands, fragments of such ligands, antibodies and small molecules that are capable of stimulating internalization of receptors and ligands/agonists. The agonists of the present invention also include labeled antibodies against target receptors which induce internalization of the receptor-agonist complex. Preferred embodiments are Somatostatin, more preferably Somatostatin-14 (Seq ID No. 5); ghrelin (Seq ID No. 6), more preferably a truncated form of ghrelin (Seq ID No. 10); Vasopressin (Seq ID No. 7); or Neurokinin 3 (Seq ID No. 8).

An agonist labeled with a fluorophore was incubated with cells expressing a target receptor, which induces internalization of the agonist-receptor complex. Then, cells were fixed and internalized agonist measured by quantifying the fluorescence intensity using suitable fluorescence imaging hardware and software, e.g. an ArrayScan 4.0 (Cellomics) with a Receptor Internalization algorithm. In the examples described below, the software used was able to identify position, size, shape and intensity of brighter objects such as nuclei, e.g. nuclei stained with a specific fluorescent stain such as Hoechst 33258, or spots, e.g. spots representing vesicles containing the fluorescent agonist. Thus, the intensity of a fluorescent agonist could be correlated to the cell numbers, as determined by stained nuclei. This allowed the determination of dose response curves and, thus, calculation of IC50 values, for compounds which inhibit internalization of fluorescently labeled agonists.

The internalization of said agonist or ligand, and a second cellular parameter (as a non limiting example, this may be the position of the cell or subpopulation of cells or cells, Ca2+ flux, expression of a reporter gene, or internalization of a second agonist etc.) are measured in said cell or cells incubated in the presence or absence of said compound.

This set-up is also useful for performing counter screenings to determine the selectivity of compounds, preferably in a single well, wherein at least two different subpopulations of cells independently express at least two receptors that can bind the same ligand. One possible non-limiting embodiment of such an assay is the use of one cell expressing one target receptor to a specific ligand, a second cell expressing a second receptor to the same ligand, wherein at least one of said cells can be recognized optically, eg. is labeled with a fluorescent or non-fluorescent dye (e.g. co-expressing GFP), or wherein the two cells can be identified based on their position. This allows for the differentiation of the two cells. Internalization of a ligand or receptor labeled with a luminophore, preferably a fluorophore, can then be detected optically and independently in the two cells. The selectivity for the two receptors of an antagonist that prevents internalization of at least one receptor can then be measured and quantified. Other cellular parameters, like, e.g. Ca2+ flux, may be correlated to internalization by parallel measurement in the multiplex method of the present invention.

Another possible set-up also useful for performing counter screenings is to determine the selectivity of compounds, preferably in single wells, wherein at least two different subpopulations of cells independently express at least two receptors that can bind the different ligands. One possible non-limiting embodiment of such an assay is the use of one cell expressing one target receptor to a specific ligand, a second cell expressing a second receptor to a second ligand, wherein said cells can be differentiated optically, e.g. by different cell size or other optical differences between the cell subpopulations, or by labeling with a luminescent or non-luminescent, dye (e.g. co-expressing GFP), or wherein the two cells can be identified based on their position. This allows for the differentiation of the two cells. Internalization of a ligand or a receptor labeled with a luminophore, preferably a fluorophore, can then be detected optically and independently in the two cells.

In another embodiment of the present invention, internalization of at least two different ligands labeled with two different labels that can be detected independently is measured in at least two different subpopulations of cells that independently express at least two different target receptors in the presence or absence of a compound.

In a preferred embodiment of the methods hereinbefore described, the target receptor is a GPCR.

In a preferred embodiment of the multiplex method hereinbefore described, at least three cellular parameters are measured.

The present invention provides the use of a luminophore-labeled agonist which induces receptor internalization, a luminophore-labeled receptor or a luminophore-labeled antibody to a GPCR for receptor-ligand tracking in multiplex high content screening of compounds. Preferably, said luminophore is a fluorophore. In one embodiment, the luminophore-labeled receptor is not a receptor to which a fluorescent protein, such as GFP, is fused.

### Description of Figures:

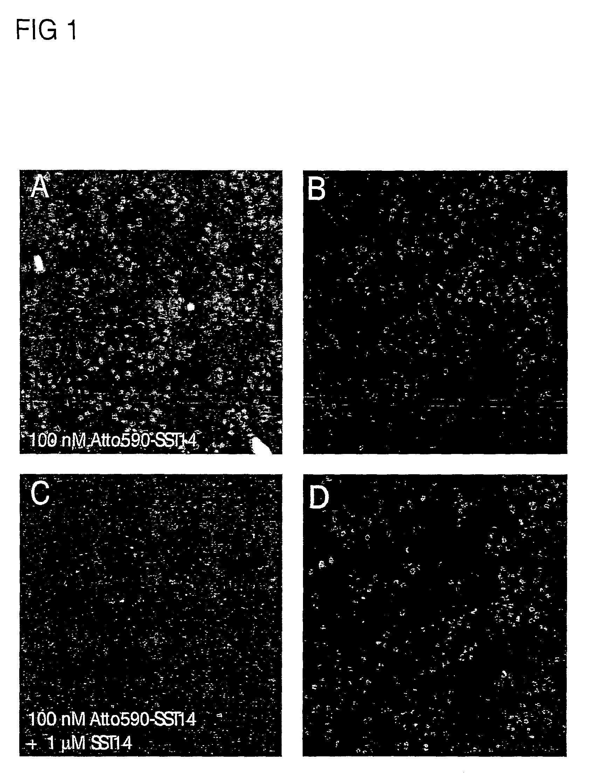

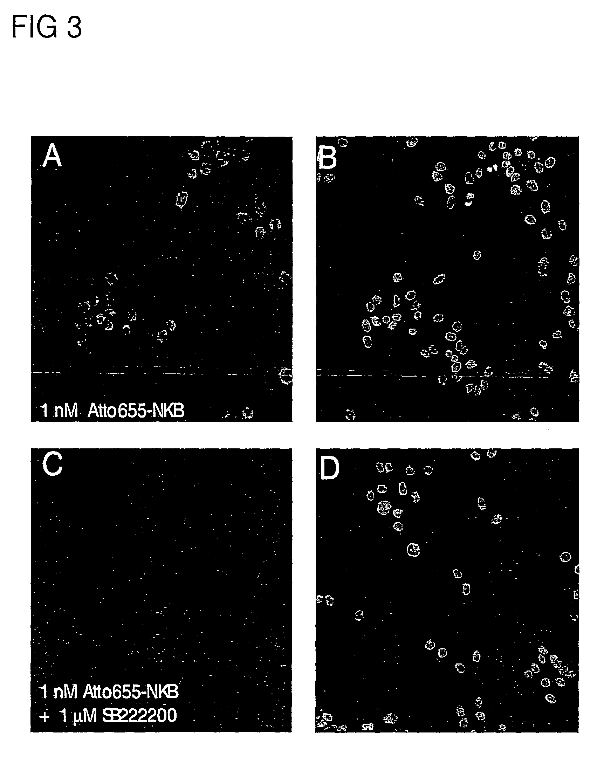

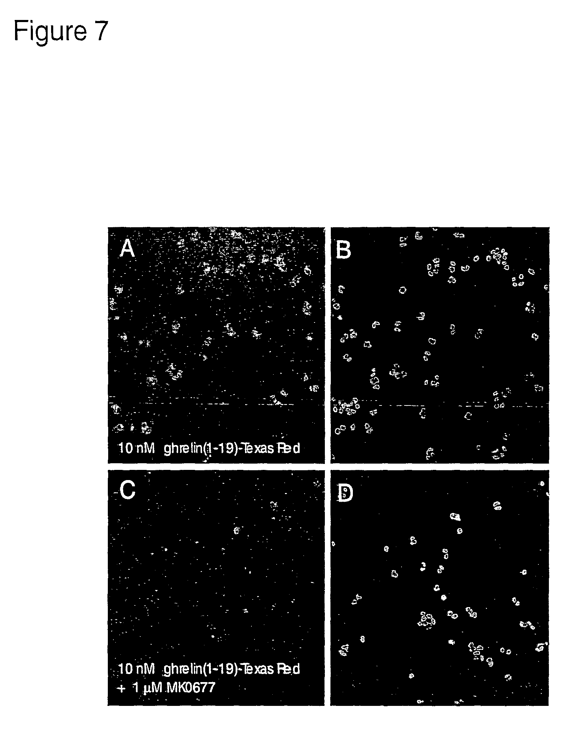

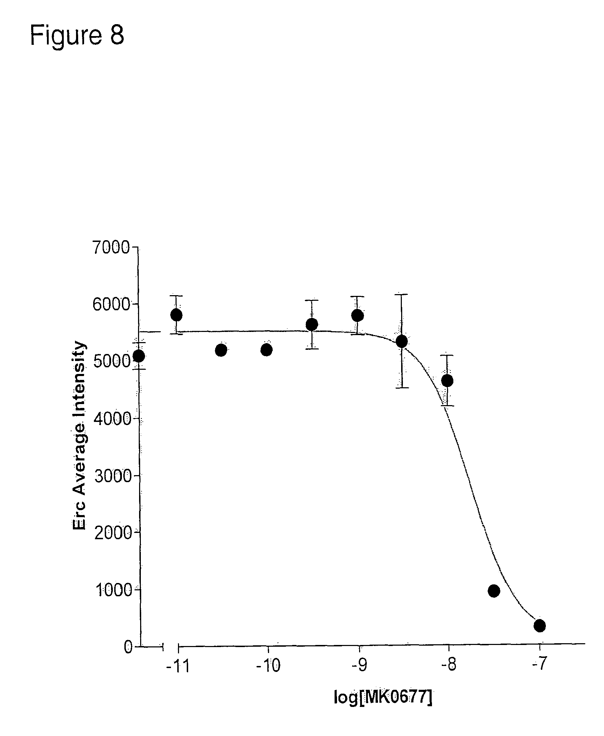

Figure 1:
   Images of fixed cell samples prepared in 96 well plate were acquired with the Cellomics ArrayScan 4.0 high content screening reader. Objective 10x. Filter setting 1: XF53-TxR, 2 s exposure time (panel A and C). Filter setting 2: XF53-hoechst, 0.1 s exposure time (panel B and D).
   A: SSTR-5 expressing cells incubated with 100 nM Atto590-SST14 for 30 minutes at 37°C, image acquired through filter setting 1. Panel B: Same sample as in A, image acquired through filter setting 2. C: SSTR-5 expressing cells incubated with 1 µM SST14 and 100 nM Atto590-SST14, image acquired through filter setting 1. D: Same sample as C, image acquired through filter setting 2.
Figure 2:
   Dose response curve
   ERC (endoplasmic recycling compartment) intensity per cell, determined from samples of SSTR-5 cells incubated with 100 nM Atto590-SST14 and varying amounts of SST14 (for sample preparation, see protocol). The analyzed images were acquired with the same optical settings as described in the legend to figure 1. Number of nuclei and intensity in ERCs was determined by use of the Receptor Internalization algorithm (Cellomics), from four images per well.
Figure 3:
   Images of fixed cell samples prepared in 96 well plate were acquired with the Cellomics ArrayScan 4.0 high content screening reader. Objective 20x. Filter setting 1: XF110-Cy5, 10 s exposure time (panel A and C). Filter setting 2: XF53-hoechst, 0.1 s exposure time (panel B and D).
   Panel A: NK3R expressing cells incubated with 1 nM Atto655-NKB for 30 minutes at 37°C, image acquired through filter setting 1. B: Same sample as A, image acquired through filter setting 2. C: NK3R expressing cells incubated with 1 µM SB222200 and 1 nM Atto655-NKB, image acquired through filter setting 1. D: Same sample as C, image acquired through filter setting 2.
Figure 4:
   Dose response curve
   ERC intensity per cell, determined from samples of NK3R cells incubated with 1 nM Atto655-NKB as a function of varying amounts of SB222200 (for sample preparation, see protocol). The analyzed images were acquired with the same optical settings as described in legend to figure 3 and analyzed by use of the Receptor internalization algorithm (Cellomics), from four image sets per well.
Figure 5:
   Mixed population of NK3R expressing cells and SSTR5 expressing cells incubated for 30 minutes at 37°C with 1 nM Atto655-NKB and 100nM Atto590-SST14 and different combinations of non-ffuorescent ligands. A,B,C: No non fluorescent ligands; D, E, F: 1 µM SST14; G, H, I: 1µM SB222200; J, K, L: 1µM SST14 and 1µM SB222200.
   Fixed cell samples prepared in 96 well plate, overlay of three images acquired with different filter settings in the Cellomics ArrayScan 4.0 high content screening reader. Objective 10x. Filter settings: XF53-TxR, 0.8 s exposure time (A, D, G, J), XF93-Cy5, 10s exposure time (B, E, H, K) and XF53-hoechst, 0.1s exposure time (C, F, I, L).
Figure 6:
   Dose response curves
   ERC intensity per cell, determined from images of samples of mixed NK3R cells and SSTR5 cells incubated with 1 nM Atto655-NKB and 100 nM Atto590-SST14. Left Y axis, filled circles: ERC intensity/cell for image pairs acquired with the XF53 hoechst/XF53-TxR filter settings. Right Y-axis, open circles: ERC intensity/cell for image pairs of the same samples acquired with the XF93-hoechst/XF93-Cy5 filter settings. Top panel: ERC intensity as a function of varying amounts of SB222200, lower panel ERC intensity as a function of varying amounts of SST14. The image sets were analyzed by use of the Receptor internalization algorithm (Cellomics), from four images per well.

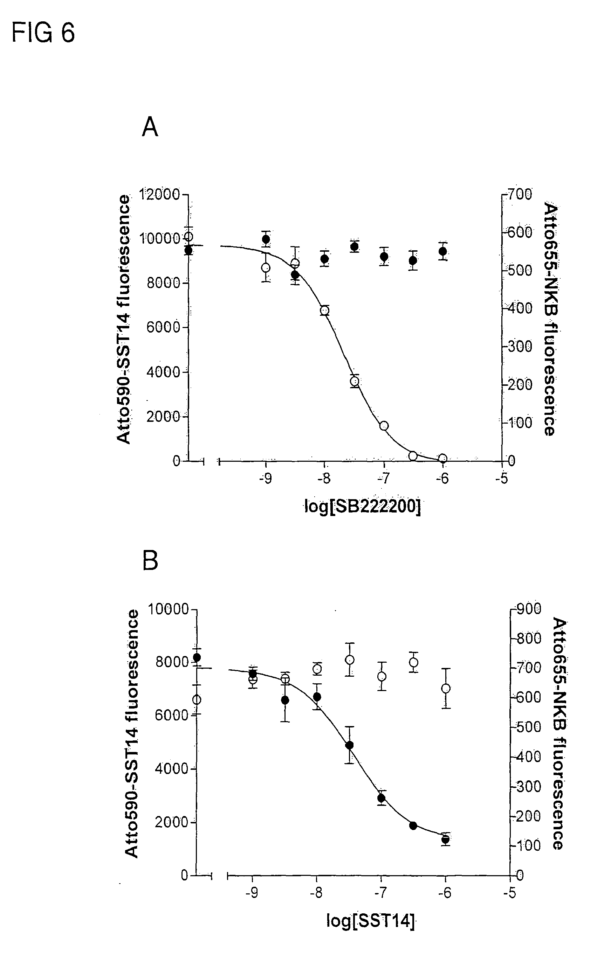

   Figure 6 presents how this setup allows simultaneous quantification in the same sample of two independent readouts for GPCR internalization.
Figure 7:
   Images of fixed cell samples prepared in 96 well plate were acquired with the Cellomics ArrayScan 4.0 high content screening reader. Objective 20x. Filter setting 1: XF53-TxR, 2 s exposure time (panel A and C). Filter setting 2: XF53-hoechst, 0.1 s exposure time (panel B and D).
   Panel A: GHSR-1a expressing cells incubated with 10 nM ghrelin(1-19)-Texas Red for 30 minutes at 37°C, image acquired through filter setting 1. B: Same sample as A, image acquired through filter setting 2. C: GHSR-1a expressing cells incubated with 1 µM MK0677 and 10 nM Ghrelin(1-19)-Texas Red, image acquired through filter setting 1. D: Same sample as C, image acquired through filter setting 2.
Figure 8:
   Dose response curve
   ERC intensity per cell, determined from samples of GHSR-1a cells incubated with 10 nM Ghrelin(1-19)-Texas Red as a function of varying amounts of MK0677 (for sample preparation, see protocol). The analyzed images were acquired with the same optical settings as described in legend to figure 7 and analyzed by use of the Receptor internalization algorithm (Cellomics), from four image sets per well.
Figure 9:
   Images of fixed cell samples prepared in 96 well plate were acquired with the Cellomics ArrayScan 4.0 high content screening reader. Objective 20x. Filter setting 1: XF93-TRITC, 2 s exposure time (panel A and C). Filter setting 2: XF93-hoechst, 0.1 s exposure time (panel B and D).
   Panel A: V1bR expressing cells incubated with 5 nM BO-TMR-vasopressin for 30 minutes at 37°C, image acquired through filter setting 1. B: Same sample as A, image acquired through filter setting 2. C: V1bR expressing cells incubated with 100 nM Arg-8-Vasopressin and BO-TMR-vasopressin, image acquired through filter setting 1. D: Same sample as C, image acquired through filter setting 2.
Figure 10:
   Dose response curve
   ERC intensity per cell, determined from samples of V1bR cells incubated with 5 nM BO-TMR-vasopressin as a function of varying amounts of Arg-8-Vasopressin (for sample preparation, see protocol). The analyzed images were acquired with the same optical settings as described in legend to figure 8 and analyzed by use of the Receptor internalization algorithm (Cellomics), from four image sets per well.

### Examples:

### Example 1: General example for agonist internalization assay

At least one agonist coupled to a fluorophore was used. A stain for nuclei may be used in addition, e.g. Hoechst 33258, to facilitate identification of individual cells. The following protocol includes the use of Hoechst 33258, however, it may be replaced by any other cellular stain, fluorescent or non-fluorescent.

The cells used for the assay may be cells naturally expressing a target receptor, or cells that are transiently or stably transfected with a target receptor.

**Plasticware & solutions**

| | |
|---|---|
| Assay plate | View plate™ 96, black; Packard Catalog nr 1104-02C, optionally poly-D lysine treated |
| 10x HBSS | GIBCO catalog No. 14065-049 |
| Hepes | 1 M solution, GIBCO catalog No. 15630-056 |
| BSA | bovine serum albumine fraction V, Sigma, catalog No. A-3059 |
| Hoechst 33258 | Sigma, catalog No. B-2261 (bisBenzimide), dissolved to 10mM in DMSO. |
| Formaldehyde | 4%, diluted from 36.5% stock (Fluka, catalog No. 47629) in PBS. |

### Sample preparation

*Day 1*: Cells were seeded 24 hours prior to the experiment:
   Cells were detached with Trypsin/EDTA. Growth medium was added and cells were resuspended by passaging through a 10 ml pipette 10-20 times. Cell concentration was measured and the cell suspension was diluted to a suitable concentration. For an experiment performed in a 96 well plate, the cells were seeded at 15.000 cells/ well in 200 µl medium. The cells were incubated at 37°C in a cell culture incubator to allow the cells to attach to the wells.
*Day* 2: I-buffer was prepared (1 x HBSS, 20 mM Hepes, 0. 1% BSA, prepared with tri-distilled water, pH not adjusted). Hoechst 33258 solution was prepared and equilibrated at 37°C. The competitor solution and the fluorescent ligand solution were prepared and equilibrated at 37°C for 5 minutes. During the following stimulation, a temperature of 37°C was maintained in the wells.

The cell-culture medium was gently aspirated. In each well, 70 µl 1.8x Hoechst solution, 10 µl fluorophore-agonist/buffer with 1% DMSO and 10 µl of increasing concentrations of fluorophore-agonist/buffer for competition, or buffer alone for control wells, were added. The mixtures were incubated for 20-40 minutes at 37°C in humidified incubator.

The cells were washed once carefully and quickly with 100 µl/well of I-buffer pre-equilibrated at 37°C. Then 100 µl/well of 4% formaldehyde (room-temperature) were added, and the wells were incubated for 15 minutes at room temperature. The solution was replaced with 150 µl PBS/well.

### Quantification of agonist internalization

Agonist internalization was quantified using an ArrayScan 4.0 from Cellomics with filters from Omega Optical, Vermont, USA. This machine is equipped with an inverted fluorescence microscope and allows automated focus on samples and acquirement of images from samples prepared in dear bottom 96 well plates. In the reader, software for image analysis is integrated, which identifies the localization of predefined types of objects.

The analysis method (algorithm) that was used here in examples 1 to 4 is called "Receptor Internalization". The analysis was based on two images acquired with different filter sets of a sample stained with a DNA-specific fluorophore and a receptor-specific fluorophore. From both images, objects were identified. From the first image, specific for the DNA stain, the number, position, size and shape of the nuclei was determined. From the second, the position, size, shape and intensity of objects consisting of concentrated receptor-specific fluorophore (so called ERCs) was identified. For the analysis of both images, certain limits for intensity, size and shape of the objects could be adjusted by the user. This allowed a selective identification and quantification of various parameters for the objects of interest.

For more details about filter settings used in the presented examples, see list at end of experimental methods.

### Analysis of data

IC50 was determined for the inhibitory effect of a non-fluorescent ligand on the receptor mediated internalization of fluorescent ligand by use of the GraphPad Prism 3.02 software from GraphPad Software, Inc.

### Example 2: Internalization of the hSSTR5 with Atto590-SST14

Protocol for preparation of fixed, double stained SSTR-5 cell populations in 96 well format, and following quantification of internalization using high content screening. Cellular stains: Hoechst 33258 (for staining of nuclei) and Atto590-SST14.

The cell line used for this assay comprised CHO cells which stably expressed the recombinant human Somatostatin receptor 5 (generated according to Rocheville et al., 2000, J. Biol. Chem. 275, 7862-7869).

As fluorescent agonist, a somatostatin peptide was used with the fluorophore Atto590 grafted to its N-terminus:

Atto590-AGC*KNFF-D-Trp-KTFTSC*-COOH (Seq ID No. 9)
* indicates formation of a cysteine bridge

The somatostatin-14 analog with the fluorophore Atto590 (Atto-tec) (Atto590-SS14) grafted to the N-terminus, was custom synthesized by Biosynthan, Berlin.
Stock Solutions
1.8 x Hoechst solution
9 µM Hoechst 33258
I-buffer
9x competitor solution
9x SST14 (Bachem catalog No. H-1490) dilution series, prepared in I-buffer.
Prepared fresh by dilution in I-buffer
9x Fluorescent ligand solution
900 nm Atto590-SST14
Prepared fresh by dilution in I-buffer
PBS

Samples were prepared as described above and in Example 1. Atto590-SST14 was used as fluorophore-agonist. Ligand internalization was quantified as described in Example 1.

Figure 1 shows images of SSTR-5 expressing cells which hd been incubated with Atto590-SST14 and in addition stained with the DNA binding fluorophore Hoechst stain. The images were acquired using the Cellomics ArrayScan Reader 4.0.

Panel A illustrates cells incubated with Atto590-SST14 acquired with filter setting selective for Atto590 fluorescence. In panel B is the image of the same sample acquired through Hoechst selective filters defining the position of the cell nuclei. In an overlay of the two images, the fluorescent ligand was visible as spots located closely around the nucleus of the cells, apparently concentrated in the endoplasmatic recycling compartments (ERCs).

The translocation of Atto590-SST14 into cytosolic compartments was to a large degree receptor specific since co-incubation with an excess of SST14 (panel C and D) block the formation of fluorescent ERCs.

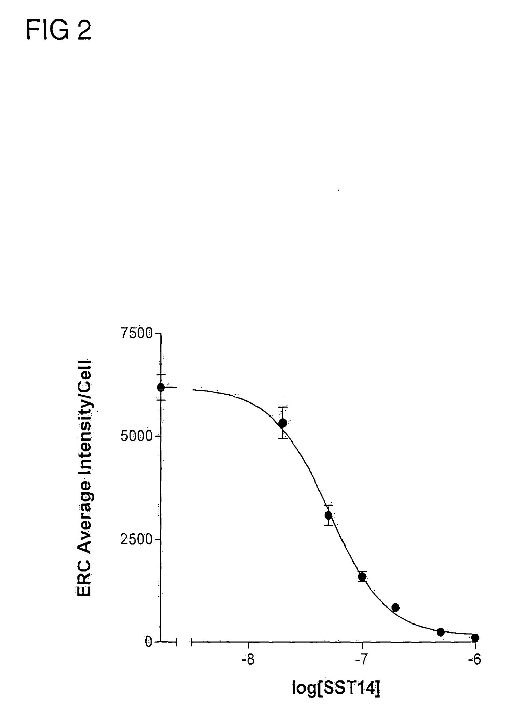

By means of a high content screening reader such as the ArrayScan 4.0 the difference in fluorescence intensity concentrated in spots could be quantified. In the present example, the algorithm for Receptor Internalization (Cellomics) was applied. This software was able to identify position, size, shape and intensity of brighter objects such as nuclei and spots in the images. Based on two images selective for respectively Hoechst and the fluorescent ligand, the algorithm identified the number of nuclei and quantify the fluorescence intensity localized in identified ERCs. Figure 2 presents how this could be used to detect the presence of a competing ligand for the receptor. Increasing concentrations of SST14 reduced in a dose dependent manner the amount of internalized Atto590-SST14 detected by the HCS reader.

IC50 values were calculated. In three independent assays, the average IC50 was determined to be 70 mM +/- 20 nM (n=3), showing that receptor pharmacology could be studied in a reproducible fashion. Filter sets are shown in Table 1.

### Example 3: Internalization of the hNK3R with Atto665-NKB

For this example, CHO cells stably expressing NK3 receptor were used (stable transfection of CHO cells with NK3 receptor is describe in Gether et al., 1992, FEBS Lett. 296,241-244)

### Cellular stain used: Hoechst 33258 (for stain of nuclei)

The preparation of samples and measurements were performed as in Example 1 with the following modifications:
As fluorescent ligand an Neurokinin B (NKB) analog was labeled with the fluorophore Atto655 (sAtto-tec, calalog No. AD 655-4).

The fluorescent NKB analog had the formula:

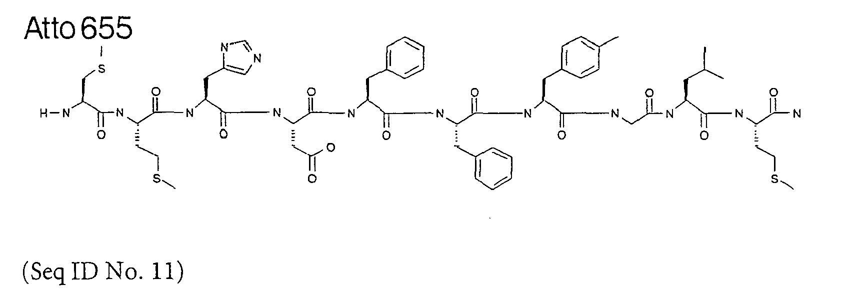

In the Neurokinin B analog, amino acid aa7 of NKB was substituted with a MethylPhenylalanine and amino acid aa1 was substituted with a Cysteine. Atto655 was grafted to the Cysteine sidechain.

The peptide was custom synthesized by Biosynthan, Berlin. The fluorophore Atto655 (Atto-tec, catalog nr AD 655-4) was covalently attached to the Cysteine side chain according to the following protocol:
The thiol-containing peptide was dissolved in 9:1 DMSO: 50 mM phosphate buffer pH 6 to a final concentration of 1-10 mM. Fluorophore reagent (10-50 mM freshly prepared solution in DMSO) was added stepwise and the mixture was left to react at room temperature for 10 minutes. The progress of the reaction was followed by analysis of samples with RP-HPLC. Finally the fluorescent labeled peptide was isolated by RP-HPLC.
   The product was found to be >90% pure by re-injection of a small sample to HPLC. The molecular weight, as determined by mass spectroscopy, was 1908.8 Da.

The fluorescent reagent had a molecular weight of 650 Da and the reactive group was maleimide. Exact structure of fluorophore was unknown. Following this information the calculated molecular mass of Atto655-NKB was 1909 Da.
Solutions
9x Fluorescent ligand solution
9 nM Atto655-NKB
Prepared fresh by dilution in I-buffer
9x competitor solution
9x dilution series, prepared in 1-buffer.
Prepared fresh by dilution in I-buffer
The assay was performed as described in Example 1. Atto655-NKB was used as fluorophore-agonist. Agonist internalization was quantified as described in Example 1.

For quantification of ligand internalization the follwing filter settings were used: Omega filters, XF93 hoechst and XF110 Cy5.

Figure 3 shows images of NK3R expressing cells which had been incubated with Atto655-NKB and Hoechst 33258 stain. The images were acquired using the Cellomics ArrayScan Reader 4.0.

Panel A illustrates cells incubated with Atto655-NKB acquired with filtersetting selective for Atto655 fluorescence. In panel B the fluorescence image of the same sample acquired through hoechst selective filters defining the position of the cell nuclei. When overlayed the fluorescent ligand was clearly visible as spots located closely around the nucleus of the cells, apparently concentrated in the endoplasmatic recycling compartments (ERCs).

The translocation of Atto655-NKB into cytosolic compartments was to a large degree receptor specific since co-incubation with an excess of the NK3R non peptide antagonist SB222200 (panel C and D) blocked the formation of fluorescent ERCs. In this example the receptor expressing cells apparently only constituted a minor part (20-30%) of the total cell-population.

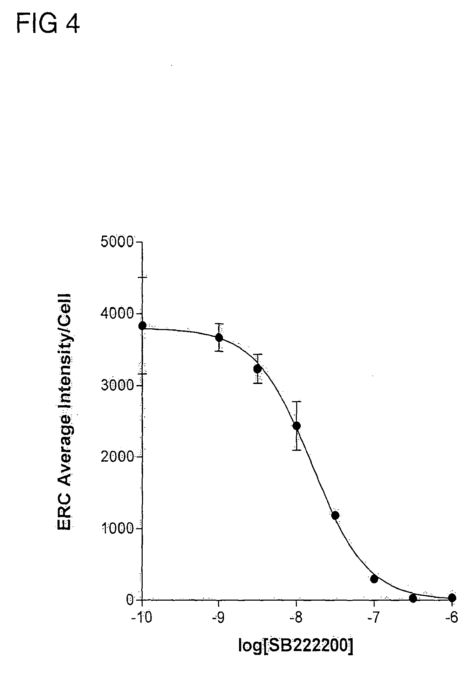

As described in Example 1, the amount of internalized ligand could be quantified using a High Content Screening reader. In this example it was demonstrated that this could be done even for cell samples where only a minor population of the cells apparently express the NK3 receptor. Figure 4 presents how increasing concentrations of SB222200 reduced in a dose dependent manner the amount of internalized Atto655-NKB detected by the HCS reader.

In two independent assays, the average IC50 was determined to be 10 nM +/- 8 nM (N=2), showing that receptor pharmacology can be studied in a reproducible fashion.

### Example 4: Internalization of the GHSR-1a with ghrelin (1-19)-Texas Red

For this example, Human Embryonic Kidney (HEK293) cells stably expressing GHSR-1a receptor were used. Cells stably expressing GHSR-1a were produced according to protocols well known in the art (see e.g. Lindemann et al., 2005, Genomics 85, 372-385; J. Sambrook, E.F. Fritsch, T. Maniatis, Molecular Cloning: a Laboratory Manual, Cold Spring Harbor Laboratory Press, New York, 1989; Casademunt, et al., EMBO J. 18 (1999) 6050-6061).

### Cellular stain used: Hoechst 33258 (for stain of nuclei)

The preparation of samples and measurements were performed as in Example 1 with the following modifications:
As fluorescent ligand was used a truncated Ghrelin analog labeled with the fluorophore Texas Red (Molecular Probes).

The fluorescent Ghrelin analog had the formula:

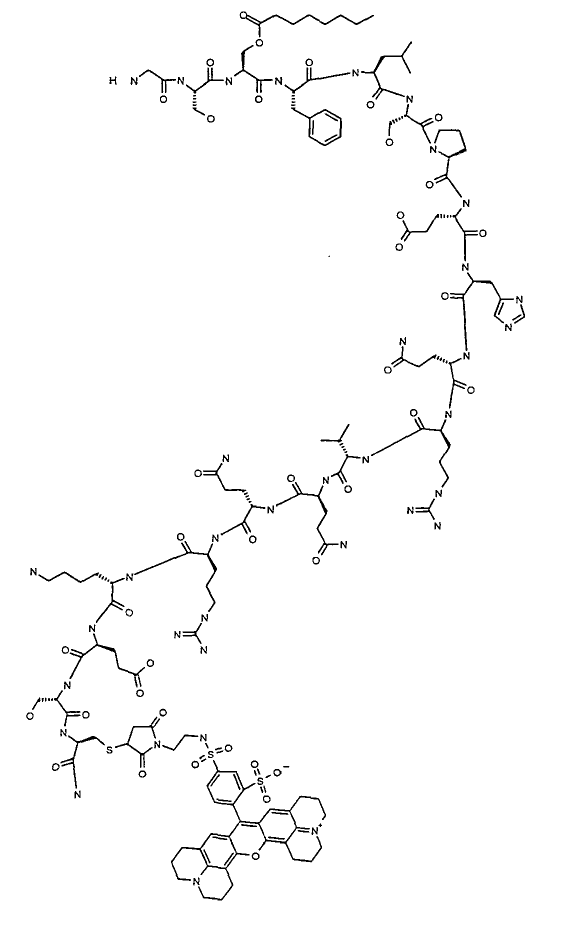

The Ghrelin analog was truncated after amino acid aa19 and the amino acid aa19 was substituted with a Cysteine (see Seq ID No. 10). The peptide was synthesized and the Texas Red was grafted to the Cysteine side chain according to the protocol described previously (see EP 1524274).

As competitor was used the nonpeptide ligand MK0677 (W094113696).
Solutions
9x Fluorescent ligand solution
90 nM Ghrelin(1-19)Texas Red
Prepared fresh by dilution in I-buffer
9x competitor solution
9x dilution series, prepared in I-buffer.
Prepared fresh by dilution in I-buffer
The assay was performed as described in Example 1. Ghrelin(1-19)-Texas Red was used as fluorophore-agonist. Agonist internalization was quantified as described in Example 1.

For quantification of ligand internalization the following filter settings were used: Omega filters, XF53 hoechst and XF53 TxR.

Figure 7 shows images of GHSR-1a expressing cells which had been incubated with ghrelin(1-19)-Texas Red and Hoechst 33258 stain. The images were acquired using the Cellomics ArrayScan Reader 4.0.

Panel A illustrates cells incubated with ghrelin(1-19)-Texas Red acquired with filter setting selective for Texas Red fluorescence. In panel B the fluorescence image of the same sample acquired through hoechst selective filters defining the position of the cell nuclei. When overlaid the fluorescent ligand was clearly visible as spots located closely around the nucleus of the cells, apparently concentrated in the endoplasmatic recycling compartments (ERCs).

The translocation of ghrelin(1-19)-Texas Red into cytosolic compartments was to a large degree receptor specific since co-incubation with an excess of the GHSR-1a non peptide antagonist MK0677 (panel C and D) blocked the formation of fluorescent ERCs.

As described in Example 1, the amount of internalized ligand could be quantified using a High Content Screening reader. In this example it was demonstrated that this could be done even for cell samples where only a minor population of the cells apparently expreseds the GHSR-1a receptor. Figure 8 presents how increasing concentrations of MK0677 reduced in a dose dependent manner the amount of internalized ghrelin(1-19)-Texas Red detected by the HCS reader.

In two independent assays, the average IC50 was determined to be 11 nM +/- 4 nM (N=4), showing that receptor pharmacology can be studied in a reproducible fashion.

### Example 5: Internalization of the hV1bR with BO-TMR-vasopressin

For this example, CHO cells stably expressing V1b receptor were used. Cells stably expressing V1b were produced according to protocols well known in the art (see e.g. Lindemann et al., 2005, Genomics 85, 372-385; J. Sambrook, E.F. Fritsch, T. Maniatis, Molecular Cloning: a Laboratory Manual, Cold Spring Harbor Laboratory Press, New York, 1989; Casademunt, et al., EMBO J. 18 (1999) 6050- 6061).

### Cellular stain used: Hoechst 33258 (for stain of nuclei)

The preparation of samples and measurements were performed as in Example 1 with the following modifications: .

As fluorescent ligand a Vasopressin analog was used, labeled with the fluorophore BODIPY-TMR (Fluorescent ligand purchased from Perkin Elmer as a part of the fluorescence polarization kit FPA120).

No sequence or structure was provided with the peptide.
As competitor, Arg-8-Vasopressin was used (Vasopressin with Arg in position 8).
The Vasopressin sequence is shown in Seq ID No. 7.
Solutions
9x Fluorescent ligand solution
5 nM BO-TMR-vasopressin
Prepared fresh by dilution in I-buffer
9x competitor solution
9x dilution series, prepared in I-buffer.
Prepared fresh by dilution in I-buffer
The assay was performed as described in Example 1. BO-TMR vasopressin was used as fluorophore-agonist. Agonist internalization was quantified as described in Example 1.

For quantification of ligand internalization the follwing filter settings were used: Omega filters, XF93 hoechst and TRITC.

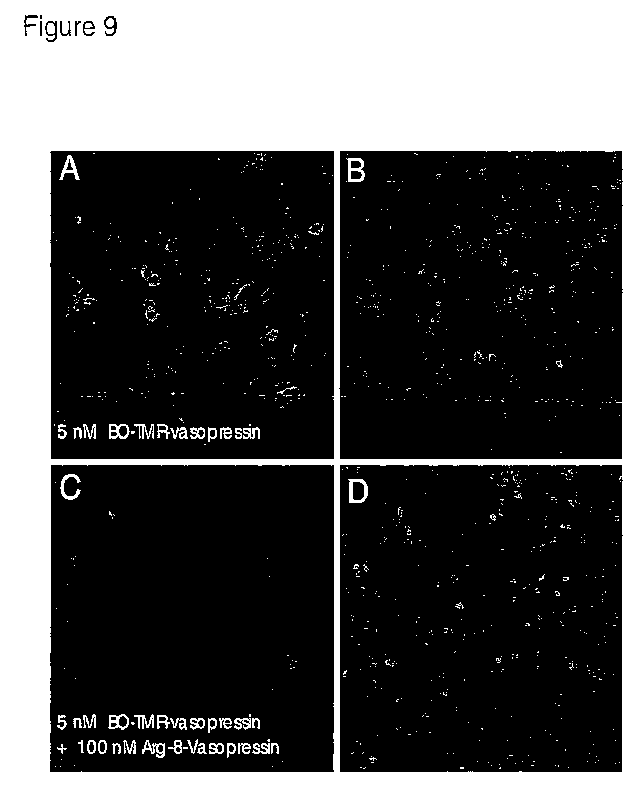

Figure 9 shows images of V1bR expressing cells which had been incubated with BO-TMR-vasopressin and Hoechst 33258 stain. The images were acquired using the Cellomics ArrayScan Reader 4.0.

Panel A illustrates cells incubated with BO-TMR-vasopressin acquired with filter setting selective for BO-TMR fluorescence. In panel B the fluorescence image of the same sample acquired through hoechst selective filters defining the position of the cell nuclei. When overlayed the fluorescent ligand was clearly visible as spots located closely around the nucleus of the cells, apparently concentrated in the endoplasmatic recycling compartments (ERCs).

The translocation of Atto655-NKB into cytosolic compartments was to a large degree receptor specific since co-incubation with an excess of the V1bR peptide agonist Arg-8-Vasopressin (panel C and D) blocked the formation of fluorescent ERCs in most cells.

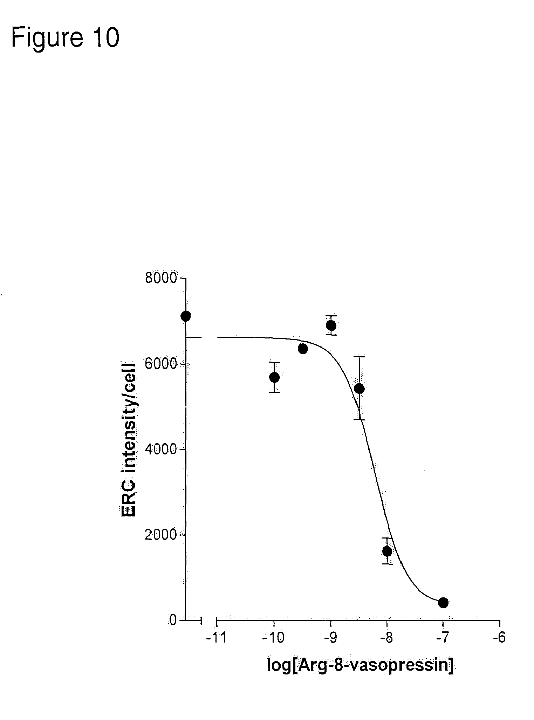

As described in Example 1, the amount of internalized ligand could be quantified using a High Content Screening reader. In this example it was demonstrated that this could be done even for cell samples where only a minor population of the cells apparently express the V1b receptor. Figure 10 presents how increasing concentrations of Arg-8-Vasopressin reduced in a dose dependent manner the amount of internalized BO-TMR-vasopressin detected by the HCS reader.

In two independent assays, the average IC50 was determined to be 5 nM and 15 nM respectively in two independent sets of experiments, showing that receptor pharmacology can be studied in a reproducible fashion.

### Example 6: Multiplexing of internalization in NK3 and SSTR5 expressing cell lines

Multiplexing describes an assay where more than one independent parameter is acquired as a readout. With High Content Screening, the image based analyzsis directly assigns a certain measured optical signal to the individual identified cell. This allows multiplexing both for multiple events within the individual cells or for events in a heterogeneous mix of cells, where subpopulations may respond differently.

In this example it was demonstrated that High Content Screening allowed multiplexing, by the mixing of two internalization assays for different GPCRs in the same well. The approach applied here was to use receptor specific fluorescent ligands with fluorescence spectra that allowed acquirement of images selective for each individual ligand.

The multiplexed assay for NK3R and SSTR-5 was performed according to examples 1 to 3 except for the co-seeding of two cell lines in the same wells and the simultaneous incubation with more than one fluorescent ligand. For the analysis, the acquirement of three rather than two images of the same field of view was necessary: One for the Hoechst stain of cell nuclei, a second one specific for Atto590 fluorescence and a third one specific for Atto655 fluorescence.

For multiplexing, the two cell lines described in Examples 2 and 3 were co-seeded for 24 h in 96 well plates as described above. The same concentrations of stock solutions as for internalization of the individual receptors were used and the volumes were adjusted to compensate for the additional competitor and fluorescent ligand solutions.

For quantification of ligand internalization with an ArrayScan 4.0 from Omega Optical, Vermont, USA, Omega filters were used. Two scans were performed on the same samples:
Scan 1: "XF93 hoechst" and "XF93 Cy5" (detection of internalized Atto655-NKB),
Scan 2: "XF53 hoechst" and "XF53 TxR" (detection of internalized Atto590-NKB).

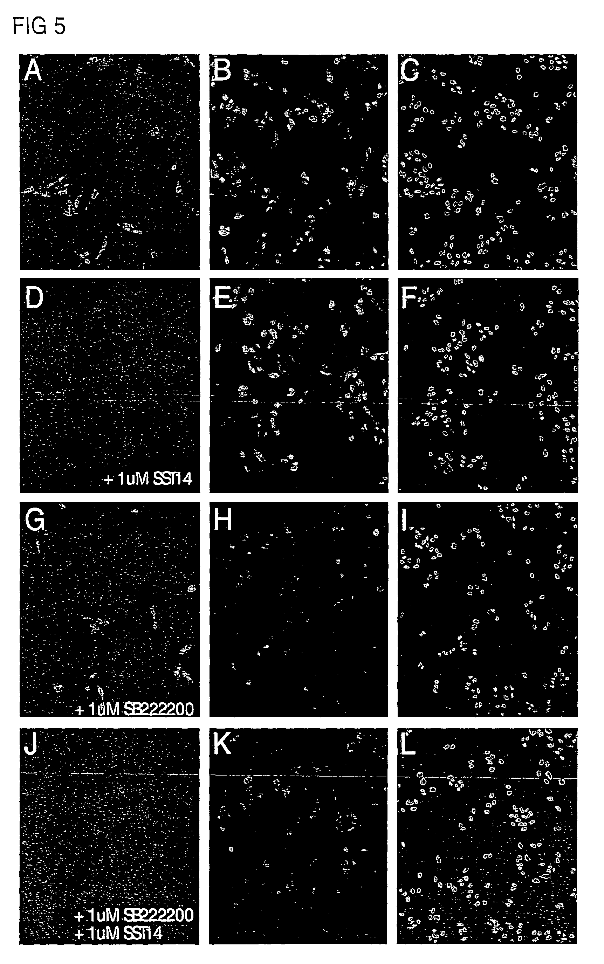

Figure 5 shows images of NK3R expressing cells mixed with SSTR-5 cells which have been incubated with Atto590-SSTR14, Atto655-NKB and Hoechst stain. The images were acquired using the Cellomics ArrayScan Reader 4.0.

Each row of images in Figure 5 was acquired for the same field of view within a sample, taken with three different filter settings which allow a virtually selective detection of the individual fluorophores Hoechst (C, F, I and L), Atto590 (B, E, H and K) and Atto655(A, D, G and J). In the first sample (images A, B and C), both the NK3R and SSTR5 expressing cells were stained with internalized ligand, respectively Atto655-NKB (A) and Atto590-SST14 (B). As expected, no significant overlap was observed between the two stains, since individual cells only expressed one of the receptors. In the image sets of the two next samples in the second and third row it was demonstrated how the receptor mediated internalization of fluorescent ligand could be blocked in a selective manner by receptor-specific non-fluorescent competitors. Thus an excess of the NK3R antagonist SB222200 had no effect on the internalization of the SSTR-5 (E) while the NK3R mediated internalization of fluorescent ligand was blocked (D). Also the SSTR-5 internalization could be inhibited in a selective manner when an excess of SST14 was added (G, H). In the last row, (J, K and L) the simultaneous blocking of NK3R and SSTR-5 internalization with a mix of the two competitors is shown.

In Figure 6, the quantification of images is presented taken of the same sample through filters as presented above which allowed a virtually selective detection of one of the fluorescent ligands. In panel A, increasing amount of SB222200 reduced the fluorescence intensity from Atto655-NKB localized in ERCs while the amount of internalized Atto590-SST14 was unaffected. The IC50 for inhibition of Atto655-NKB internalization was determined to 21 nM which corresponds well with the IC50 found for the individual NK3 ligand tracking system as presented in example 2. In B it was the inverse situation, where the ERC intensity of Atto590-SST14 was reduced by SST14, while the intensity of Atto655-NKB in ERCs was unchanged. The IC50 at 36 nM for inhibition of Atto590-SST14 internalization was in good correlation with the value found for the SSTR-5 system as presented in example 2.

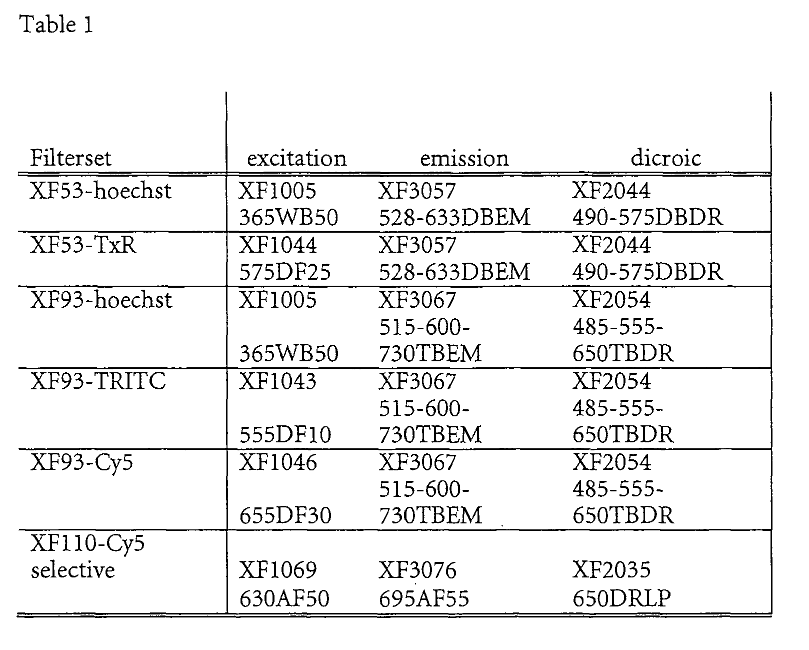

## Claims

1. A multiplex method for high content screening of compounds, wherein at least one cellular parameter of at least one single cell or at least one subpopulation of cells or a heterogeneous population of cells which express at least one target receptor, are measured optically in the presence or absence of a compound.

2. The method of claim 1, wherein at least two cellular parameters are measured.

3. The method of claim 1, wherein at least three cellular parameters are measured.

4. The method of any one of claims 2 to 3, wherein at least two single cells or two subpopulations of cells which express different target receptors are measured.

5. The multiplex method of any one of claims 1 to 4, wherein at least one of the cellular parameters is tracking of ligand-receptor complexes, and wherein said ligand-receptor complexes are tracked by optical detection of a labeled ligand or labeled receptor.

6. The multiplex method of any one of claims 1 to 5, wherein at least one of the cellular parameters is tracking of ligand-receptor internalization, and wherein said ligand-receptor internalization is tracked by optical detection of a labeled ligand or labeled receptor.

7. The multiplex method of any one of claims 5 or 6, wherein said labeled receptor does not include a receptor to which a fluorescent protein is fused.

8. The multiplex method of any one of claims 5 to 7, wherein said labeled ligand is a ligand to which a luminophore is grafted.

9. The multiplex method of any one of claims 5 to 8, wherein said labeled receptor is a receptor which is bound by a specific antibody, and wherein said antibody is labeled with a label that can be detected optically.

10. The multiplex method of any one of claims 2 to 9, wherein the different cells or subpopulations of cells independently express different target receptors that can bind the same ligand.

11. The multiplex method of any one of claims 6 to 9, wherein internalization of at least two different ligands labeled with two different labels that can be detected independently is measured in at least two different subpopulations of cells that independently express at least two different target receptors in the presence or absence of a compound.

12. The multiplex screening method of any one of claims 1 to 11, wherein at least one target receptor is a GPCR.

13. The multiplex screening method of any one of claims 1 to 12, wherein at least one label is a fluorophore.

14. Use of a luminophore- labeled agonist which induces receptor internalization for receptor-ligand tracking in multiplex high content screening of compounds.

15. Use of a luminophore-labeled receptor for receptor-ligand tracking in multiplex high content screening of compounds.

16. Use of a luminophore-labeled antibody to a GPCR for receptor-ligand tracking in multiplex high content screening of compounds.

17. The methods and uses hereinbefore described, especially with reference to the foregoing examples.


SEQUENCE LISTING

<110> F. Hoffmann-La Roche AG

<120> Internalization assays

<130> 22817

<160> 11

<170> PatentIn version 3.2

<210> 1

<211> 364

<212> PRT

<213> Homo sapiens

<220>

<221> Somatostatin receptor 5

<222> (1)..(364)

<223> SSTR 5; LocusID:6755

<400> 1

Met Glu Pro Leu Phe Pro Ala Ser Thr Pro Ser Trp Asn Ala Ser Ser

1               5                   10                  15

Pro Gly Ala Ala Ser Gly Gly Gly Asp Asn Arg Thr Leu Val Gly Pro

            20                  25                  30

Ala Pro Ser Ala Gly Ala Arg Ala Val Leu Val Pro Val Leu Tyr Leu

        35                  40                  45

Leu Val Cys Ala Ala Gly Leu Gly Gly Asn Thr Leu Val Ile Tyr Val

    50                  55                  60

Val Leu Arg Phe Ala Lys Met Lys Thr Val Thr Asn Ile Tyr Ile Leu

65                  70                  75                  80

Asn Leu Ala Val Ala Asp Val Leu Tyr Met Leu Gly Leu Pro Phe Leu

                85                  90                  95

Ala Thr Gln Asn Ala Ala Ser Phe Trp Pro Phe Gly Pro Val Leu Cys

            100                 105                 110

Arg Leu Val Met Thr Leu Asp Gly Val Asn Gln Phe Thr Ser Val Phe

        115                 120                 125

Cys Leu Thr Val Met Ser Val Asp Arg Tyr Leu Ala Val Val His Pro

    130                 135                 140

Leu Ser Ser Ala Arg Trp Arg Arg Pro Arg Val Ala Lys Leu Ala Ser

145                 150                 155                 160

Ala Ala Ala Trp Val Leu Ser Leu Cys Met Ser Leu Pro Leu Leu Val

                165                 170                 175

Phe Ala Asp Val Gln Glu Gly Gly Thr Cys Asn Ala Ser Trp Pro Glu

            180                 185                 190

Pro Val Gly Leu Trp Gly Ala Val Phe Ile Ile Tyr Thr Ala Val Leu

        195                 200                 205

Gly Phe Phe Ala Pro Leu Leu Val Ile Cys Leu Cys Tyr Leu Leu Ile

    210                 215                 220

Val Val Lys Val Arg Ala Ala Gly Val Arg Val Gly Cys Val Arg Arg

225                 230                 235                 240

Arg Ser Glu Arg Lys Val Thr Arg Met Val Leu Val Val Val Leu Val

                245                 250                 255

Phe Ala Gly Cys Trp Leu Pro Phe Phe Thr Val Asn Ile Val Asn Leu

            260                 265                 270

Ala Val Ala Leu Pro Gln Glu Pro Ala Ser Ala Gly Leu Tyr Phe Phe

        275                 280                 285

Val Val Ile Leu Ser Tyr Ala Asn Ser Cys Ala Asn Pro Val Leu Tyr

    290                 295                 300

Gly Phe Leu Ser Asp Asn Phe Arg Gln Ser Phe Gln Lys Val Leu Cys

305                 310                 315                 320

Leu Arg Lys Gly Ser Gly Ala Lys Asp Ala Asp Ala Thr Glu Pro Arg

                325                 330                 335

Pro Asp Arg Ile Arg Gln Gln Gln Glu Ala Thr Pro Pro Ala His Arg

            340                 345                 350

Ala Ala Ala Asn Gly Leu Met Gln Thr Ser Lys Leu

        355                 360

<210> 2

<211> 366

<212> PRT

<213> Homo sapiens

<220>

<221> Growth Hormone Secretagogue Receptor 1a

<222> (1)..(366)

<223> GHSR 1a; LocusID:2693

<400> 2

Met Trp Asn Ala Thr Pro Ser Glu Glu Pro Gly Phe Asn Leu Thr Leu

1               5                   10                  15

Ala Asp Leu Asp Trp Asp Ala Ser Pro Gly Asn Asp Ser Leu Gly Asp

            20                  25                  30

Glu Leu Leu Gln Leu Phe Pro Ala Pro Leu Leu Ala Gly Val Thr Ala

        35                  40                  45

Thr Cys Val Ala Leu Phe Val Val Gly Ile Ala Gly Asn Leu Leu Thr

    50                  55                  60

Met Leu Val Val Ser Arg Phe Arg Glu Leu Arg Thr Thr Thr Asn Leu

65                  70                  75                  80

Tyr Leu Ser Ser Met Ala Phe Ser Asp Leu Leu Ile Phe Leu Cys Met

85                  90                  95

Pro Leu Asp Leu Val Arg Leu Trp Gln Tyr Arg Pro Trp Asn Phe Gly

100                 105                 110

Asp Leu Leu Cys Lys Leu Phe Gln Phe Val Ser Glu Ser Cys Thr Tyr

115                 120                 125

Ala Thr Val Leu Thr Ile Thr Ala Leu Ser Val Glu Arg Tyr Phe Ala

130                 135                 140

Ile Cys Phe Pro Leu Arg Ala Lys Val Val Val Thr Lys Gly Arg Val

145                 150                 155                 160

Lys Leu Val Ile Phe Val Ile Trp Ala Val Ala Phe Cys Ser Ala Gly

165                 170                 175

Pro Ile Phe Val Leu Val Gly Val Glu His Glu Asn Gly Thr Asp Pro

180                 185                 190

Trp Asp Thr Asn Glu Cys Arg Pro Thr Glu Phe Ala Val Arg Ser Gly

195                 200                 205

Leu Leu Thr Val Met Val Trp Val Ser Ser Ile Phe Phe Phe Leu Pro

210                 215                 220

Val Phe Cys Leu Thr Val Leu Tyr Ser Leu Ile Gly Arg Lys Leu Trp

225                 230                 235                 240

Arg Arg Arg Arg Gly Asp Ala Val Val Gly Ala Ser Leu Arg Asp Gln

                245                 250                 255

Asn His Lys Gln Thr Val Lys Met Leu Ala Val Val Val Phe Ala Phe

            260                 265                 270

Ile Leu Cys Trp Leu Pro Phe His Val Gly Arg Tyr Leu Phe Ser Lys

        275                 280                 285

Ser Phe Glu Pro Gly Ser Leu Glu Ile Ala Gln Ile Ser Gln Tyr Cys

    290                 295                 300

Asn Leu Val Ser Phe Val Leu Phe Tyr Leu Ser Ala Ala Ile Asn Pro

305                 310                 315                 320

Ile Leu Tyr Asn Ile Met Ser Lys Lys Tyr Arg Val Ala Val Phe Arg

                325                 330                 335

Leu Leu Gly Phe Glu Pro Phe Ser Gln Arg Lys Leu Ser Thr Leu Lys

            340                 345                 350

Asp Glu Ser Ser Arg Ala Trp Thr Glu Ser Ser Ile Asn Thr

        355                 360                 365

<210> 3

<211> 424

<212> PRT

<213> Homo sapiens

<220>

<221>  Vasopressin receptor 1B

<222>  (1)..(424)

<223>  LocusID:553

<400>  3

Met Asp Ser Gly Pro Leu Trp Asp Ala Asn Pro Thr Pro Arg Gly Thr

1               5                   10                  15

Leu Ser Ala Pro Asn Ala Thr Thr Pro Trp Leu Gly Arg Asp Glu Glu

            20                  25                  30

Leu Ala Lys Val Glu Ile Gly Val Leu Ala Thr Val Leu Val Leu Ala

        35                  40                  45

Thr Gly Gly Asn Leu Ala Val Leu Leu Thr Leu Gly Gln Leu Gly Arg

    50                  55                  60

Lys Arg Ser Arg Met His Leu Phe Val Leu His Leu Ala Leu Thr Asp

65                  70                  75                  80

Leu Ala Val Ala Leu Phe Gln Val Leu Pro Gln Leu Leu Trp Asp Ile

                85                  90                  95

Thr Tyr Arg Phe Gln Gly Pro Asp Leu Leu Cys Arg Ala Val Lys Tyr

            100                 105                 110

Leu Gln Val Leu Ser Met Phe Ala Ser Thr Tyr Met Leu Leu Ala Met

        115                 120                 125

Thr Leu Asp Arg Tyr Leu Ala Val Cys His Pro Leu Arg Ser Leu Gln

    130                 135                 140

Gln Pro Gly Gln Ser Thr Tyr Leu Leu Ile Ala Ala Pro Trp Leu Leu

145                 150                 155                 160

Ala Ala Ile Phe Ser Leu Pro Gln Val Phe Ile Phe Ser Leu Arg Glu

                165                 170                 175

Val Ile Gln Gly Ser Gly Val Leu Asp Cys Trp Ala Asp Phe Gly Phe

            180                 185                 190

Pro Trp Gly Pro Arg Ala Tyr Leu Thr Trp Thr Thr Leu Ala Ile Phe

        195                 200                 205

Val Leu Pro Val Thr Met Leu Thr Ala Cys Tyr Ser Leu Ile Cys His

    210                 215                 220

Glu Ile Cys Lys Asn Leu Lys Val Lys Thr Gln Ala Trp Arg Val Gly

225                 230                 235                 240

Gly Gly Gly Trp Arg Thr Trp Asp Arg Pro Ser Pro Ser Thr Leu Ala

                245                 250                 255

Ala Thr Thr Arg Gly Leu Pro Ser Arg Val Ser Ser Ile Asn Thr Ile

            260                 265                 270

Ser Arg Ala Lys Ile Arg Thr Val Lys Met Thr Phe Val Ile Val Leu

        275                 280                 285

Ala Tyr Ile Ala Cys Trp Ala Pro Phe Phe Ser Val Gln Met Trp Ser

    290                 295                 300

Val Trp Asp Lys Asn Ala Pro Asp Glu Asp Ser Thr Asn Val Ala Phe

305                 310                 315                 320

Thr Ile Ser Met Leu Leu Gly Asn Leu Asn Ser Cys Cys Asn Pro Trp

                325                 330                 335

Ile Tyr Met Gly Phe Asn Ser His Leu Leu Pro Arg Pro Leu Arg His

            340                 345                 350

Leu Ala Cys Cys Gly Gly Pro Gln Pro Arg Met Arg Arg Arg Leu Ser

        355                 360                 365

Asp Gly Ser Leu Ser Ser Arg His Thr Thr Leu Leu Thr Arg Ser Ser

    370                 375                 380

Cys Pro Ala Thr Leu Ser Leu Ser Leu Ser Leu Thr Leu Ser Gly Arg

385                 390                 395                 400

Pro Arg Pro Glu Glu Ser Pro Arg Asp Leu Glu Leu Ala Asp Gly Glu

                405                 410                 415

Gly Thr Ala Glu Thr Ile Ile Phe

            420

<210>  4

<211>  465

<212>  PRT

<213>  Homo sapiens

<220>

<221>  Neurokinin 3 receptor

<222>  (1)..(465)

<223>  NK3R; LocusID:6870

<400>  4

<210> 5

<211> 116

<212> PRT

<213> Homo sapiens

<220>

<221> somatostatin 14

<222> (1)..(116)

<223> LocusID:6750

<400> 5

Met Leu Ser Cys Arg Leu Gln Cys Ala Leu Ala Ala Leu Ser Ile Val

1               5                   10                  15

Leu Ala Leu Gly Cys Val Thr Gly Ala Pro Ser Asp Pro Arg Leu Arg

            20                  25                  30

Gln Phe Leu Gln Lys Ser Leu Ala Ala Ala Ala Gly Lys Gln Glu Leu

        35                  40                  45

Ala Lys Tyr Phe Leu Ala Glu Leu Leu Ser Glu Pro Asn Gln Thr Glu

    50                  55                  60

Asn Asp Ala Leu Glu Pro Glu Asp Leu Ser Gln Ala Ala Glu Gln Asp

65                  70                  75                  80

Glu Met Arg Leu Glu Leu Gln Arg Ser Ala Asn Ser Asn Pro Ala Met

                85                  90                  95

Ala Pro Arg Glu Arg Lys Ala Gly Cys Lys Asn Phe Phe Trp Lys Thr

            100                 105                 110

Phe Thr Ser Cys

        115

<210>  6

<211>  117

<212>  PRT

<213>  Homo sapiens

<220>

<221>  ghrelin precursor

<222>  (1)..(117)

<223>  LocusID:51738

<400>  6

Met Pro Ser Pro Gly Thr Val Cys Ser Leu Leu Leu Leu Gly Met Leu

1               5                   10                  15

Trp Leu Asp Leu Ala Met Ala Gly Ser Ser Phe Leu Ser Pro Glu His
            20                  25                  30
Gln Arg Val Gln Gln Arg Lys Glu Ser Lys Lys Pro Pro Ala Lys Leu
        35                  40                  45
Gln Pro Arg Ala Leu Ala Gly Trp Leu Arg Pro Glu Asp Gly Gly Gln
    50                  55                  60
Ala Glu Gly Ala Glu Asp Glu Leu Glu Val Arg Phe Asn Ala Pro Phe
65                  70                  75                  80
Asp Val Gly Ile Lys Leu Ser Gly Val Gln Tyr Gln Gln His Ser Gln
                85                  90                  95
Ala Leu Gly Lys Phe Leu Gln Asp Ile Leu Trp Glu Glu Ala Lys Glu
            100                 105                 110
Ala Pro Ala Asp Lys
        115

<210>  7
<211>  164
<212>  PRT
<213>  Homo sapiens
<220>

<221>  vasopressin (avp)

<222>  (1)..(164)

<223>  LocusID: 551

<400>  7

<210>  8

<211>  121

<212>  PRT

<213>  Homo sapiens

<220>

<221>  Neurokinin B

<222>  (1)..(121)

<223>  LocusID:6866

<400>  8

<210> 9

<211> 14

<212> PRT

<213> Homo sapiens

<220>

<221> somatostatin 14

<222> (1)..(14)

<223> C-terminal somatostatin peptide (103-117)

disulfide bond between C(3) and C(14)

<220>

<221> somatostatin 14

<222>  (8)..(8)

<223>  D-Trp

<400>  9

Ala Gly Cys Lys Asn Phe Phe Trp Lys Thr Phe Thr Ser Cys

1               5                   10

<210>  10

<211>  19

<212>  PRT

<213>  Homo sapiens

<220>

<221>  ghrelin(1-19)

<222>  (1)..(19)

<220>

<221>  ghrelin(1-19)

<222>  (3)..(3)

<223>  octanoyl derivative or Dapa-octanoyl derivative

<400>  10

Gly Ser Ser Phe Leu Ser Pro Glu His Asn Arg Val Asn Asn Arg Lys

1               5                   10                  15

Glu Ser Cys

<210> 11

<211> 10

<212> PRT

<213> Homo sapiens

<220>

<221> Neurokinin B peptide

<222> (1)..(10)

<220>

<221> Neurokinin B peptide

<222> (7)..(7)

<223> Phe(7)=Methyl-Phenylalanine

<400> 11

Cys Met His Asp Phe Phe Phe Gly Leu Met

1               5                   10